# EUROPÄISCHE PATENTSCHRIFT

(11) **EP 2 081 609 B1**
(45) Veröffentlichungstag und Bekanntmachung des Hinweises auf die Patenterteilung: **21.03.2012**
(21) Anmeldenummer: 07818767.1
(22) Anmeldetag: 06.10.2007
(51) Int. Cl.: A61L 15/58, C09J 123/02, B32B 7/12, C09J 123/08, C09J 123/10

(54) **HEISSSCHMELZKLEBEMASSE**
HOT-MELT ADHESIVE SUBSTANCE
SUBSTANCE ADHÉSIVE THERMOFUSIBLE

(30) Priorität: 18.10.2006 DE 102006049089
(43) Veröffentlichungstag der Anmeldung: 29.07.2009
(73) Patentinhaber: Clariant Finance (BVI) Limited, Road Town, Tortola (VG)
(72) Erfinder: BACH, Sebastijan, 86462 Achsheim (DE); HOHNER, Gerd, 86368 Gersthofen (DE)
(74) Vertreter: Jacobi, Carola
(86) Internationale Anmeldenummer: PCT/EP2007/008691
(87) Internationale Veröffentlichungsnummer: WO 2008/046536

(56) Entgegenhaltungen:
- EP-A- 1 645 608
- WO-A-02/45763
- WO-A-2004/104128
- DE-A1- 19 648 895
- US-A1- 2005 054 779

## Beschreibung

Die Erfindung betrifft die Verwendung einer Heißschmelzklebemasse zum Verkleben von faserigen Materialien wie Wirrfaservliese oder gewobene Textilien mit glatten Substratoberflächen wie Folien aus Kunststoff oder Metall.

Zahlreiche Hygieneartikel, insbesondere Höschenwindeln und Slipeinlagen, aber auch spezielle Tücher zum Reinigen und Desinfizieren sind entsprechend ihren bestimmungsgemäßen Verwendungszwecken aus unterschiedlichen, mehrlagigen Materialien aufgebaut. So bestehen Höschenwindeln aus einer für Flüssigkeiten gut durchlässigen inneren Schicht, dann einer faserigen inneren Schicht, beispielsweise einem Vlies, die Absorbermaterial wie Superabsorber enthält, und abschließend einer für Wasser und Flüssigkeiten aller Art undurchlässigen äußeren Folie aus Kunststoff. Die mehrlagigen Materialien müssen dabei so fest miteinander verbunden werden, dass sie bei ihrem bestimmungsgemäßen Einsatzzweck einerseits nicht sogleich delaminieren oder verrutschen und dass sie andererseits auch einen hinreichend guten Tragekomfort bieten. Weil die betreffenden Hygieneartikel bei Gebrauch überwiegend in unmittelbarem Kontakt zu solchen Körperpartien stehen, an denen ihr Träger oder ihre Trägerin besonders empfindlich gegenüber äußeren Reizen ist, dürfen sie keine starren oder spitzen Halteelemente aufweisen, die theoretisch zu Hautverletzungen führen können. Weiter dürfen auch keine unnachgiebigen, harten Verbindungsnähte vorhanden sein, die Druck auf die Haut ausüben oder Reibung erzeugen.

Unter diesen Umständen werden an die Klebematerialien, insbesondere die Klebematerialien zum Verkleben der Vliesoberfläche mit der wasserundurchlässigen Folie, hohe Anforderungen gestellt. So muss die Verklebung auch bei Temperaturbelastung sehr gut und dauerbeständig fest sein, sie muss starken mechanischen Belastungen standhalten und sie darf nicht wasserempfindlich sein. Die Elastizität und Beweglichkeit der unterschiedlichen Materialien im Hygieneartikel sowie deren anwendungstechnische Eigenschaften dürfen durch die Anwesenheit der Klebemasse nicht beeinträchtigt werden. Aus toxikologischen und ökotoxikologischen Gründen werden lösungsmittelfreie Klebematerialien gefordert, die in sehr dünnen Schichten aufgetragen oder aufgesprüht werden können und bereits in sehr kleinen Gewichtsmengen hohe Klebekräfte aufweisen. So soll das Anfertigen besonders leichter Artikel mit gutem Tragekomfort ermöglicht werden.

Stand der Technik sind Heißschmelzklebemassen enthaltend Styrol-Isoprenhaltige oder Styrol-Butadien-haltige Blockcopolymere, beispielsweise unter dem Handelsnamen KRATON erhältliche hydrierte Styrol-Butadien-Blockcopolymere. Styrol-Blockcopolymere sind als solche ohne zusätzliche Klebekomponenten nicht klebrig. Um die Klebrigkeit und eine für die Anwendung erforderlich Plastizität zu erreichen, werden den styrolhaltigen Blockcopolymeren Harze oder Öle zugesetzt. Diese Zusätze sind permanent klebrig und für den Anwender schwierig zu handhaben. Zudem neigen sie dazu, das Vliesgewebe zu durchdringen, was zu farbigen Flecken und zu einem weniger günstigen optischen Erscheinungsbild der Hygieneartikel führen kann. Weiter sind bei Verwendung solcher Heißschmelzklebemassen dickere Vliesmaterialien erforderlich, was dem Verbraucherwunsch nach dünnen und leichten Hygieneartikeln entgegensteht.

In WO 01/14487 werden Heißschmelzklebemassen und deren Verwendung als Konstruktionskleber bei der Herstellung von Babywindeln beschrieben. Die Heißschmelzklebemassen enthalten in Gegenwart von Metallocen als Katalysator hergestellte Copolymere aus Ethylen und C₃ bis C₂₀-alpha-Olefinen, insbesondere Ethylene-Buten-1-Copolymere, wie sie etwa unter dem Handelsnamen EXACT, oder Ethylen-Octen-1-Copolymere, wie sie unter den Handelsnamen AFFINITY und ENGAGE erhältlich sind. Die Adhäsionskräfte der dort beschriebenen Klebemassen sind allerdings noch verbesserungswürdig.

Die DE 10 2004 048 536 und die DE 103 23 617 beschreiben bereits Heißschmelzmassen und deren Verwendung, die Polyolefinwachse enthalten, die mit Hilfe von Metallocenverbindungen als Katalysatoren hergestellt sind. Daraus war auch bekannt, dass solche Polyolefinwachse Glasübergangstemperaturen T_{g} von kleiner/gleich -10 °C besitzen.

Aufgabe der vorliegenden Erfindung war es daher, eine neue Verwendung für eine Heißschmelzklebemasse zu entwickeln, die lösungsmittelfrei, geruchlos und toxikologisch wie ökotoxikologisch unbedenklich ist. Die verwendete Heißschmelzklebemasse sollte bei Temperaturen von 150 °C eine Viskosität unter 15 000 mPa·s aufweisen und bei Temperaturen im Bereich von 100 bis 180 °C sprühbar und als sehr dünner Film gleichmäßig auftragbar sein, ohne dabei Vliesmaterial vollständig zu durchdringen. Verwendungsgemäß erwünscht ist weiter eine Heißschmelzklebemasse, die bereits bei einer Gewichtsmenge von weniger als 6 g/m² ihre volle Klebewirkung entfaltet und dabei sehr gute Adhäsionskraft entwickelt.

Gelöst wird diese Aufgabe durch einer Heißschmelzklebemasse der eingangs genannten Art, deren Kennzeichenmerkmal darin zu sehen ist, dass sie mindestens ein Polyolefin enthält, das durch Polymerisation in Gegenwart von Metallocen als Katalysator hergestellt wurde und das einen Erweichungspunkt Ring/Kugel zwischen 50 und 165 °C sowie eine Schmelzviskosität, gemessen bei einer Temperatur von 170 °C, zwischen 20 und 40 000 mPa·s aufweist.

Überraschend wurde gefunden, dass sich die erfindungsgemäß verwendete Heißschmelzklebemasse dann in besonders vorteilhafter Weise als Heißschmelzkleber zum Verkleben von Fasermaterial wie Vliesmaterial mit Folien und zum Laminieren von Hygieneartikeln eignet, wenn sie ein oder mehrere Polyolefin/e enthält, die eine Glasübergangstemperatur Tg von maximal -10 °C und einen Schmelzflussindex MFI von mehr als 30 g/10 min aufweist, gemessen nach ISO 1133 bei einer Temperatur von 190 °C und einem Auflagegewicht von 2,16 kg.

Weiterhin wurde gefunden, dass die erfindungsgemäß verwendete Heizschmelzklebemasse vorteilhaft auch für die Verklebung von textilen Materialien, z.B. Geweben eingesetzt werden kann.

Die erfindungsgemäß verwendete Heißschmelzklebemasse weist überragende Eigenschaften in Bezug auf ihre Klebekraft zu den jeweiligen Substraten auch bei Temperaturen im Bereich von 37 bis 50 °C auf und sie kann vor allem bei Viskositäten im Bereich von 200 bis 10 000 mPa·s bei 150 °C sehr dünn auf Oberflächen aufgesprüht werden. Sie zeigt überraschend keinerlei Tendenz, faseriges Material wie Vliesmaterial oder textile Schichten zu durchdringen. Die erfindungsgemäß verwendete Heißschmelzklebemasse ist nicht permanent klebrig, sondern erst bei Verarbeitung aus der Schmelze, und somit für den Anwender bei der Fertigung von Hygieneartikeln aller Art leicht handhabbar.

Die erfindungsgemäß verwendete Heißschmelzklebemasse zeichnet sich durch eine gute innere Elastizität aus, die auch bei tiefen Temperaturen erhalten bleibt und den vielfachen Verformungen der mit ihr beklebten Vlies- und Folienmaterialien problemlos standhält. Sie ist wasserunlöslich, so dass es in Anwesenheit von Wasser oder hoher Luftfeuchtigkeit nicht zu ungewollten Ablöseerscheinungen kommen kann.

Vorteilhaft ist auch die bei 100 bis 180 °C vergleichsweiser geringer Viskosität der erfindungsgemäßen Heißschmelzklebemasse, die besonders niedrigere Verarbeitungstemperaturen ermöglicht. Schrumpfungen oder Schwindungseffekte, die bei hohen Verarbeitungstemperaturen immer wieder leicht auftreten können, werden somit vermieden.

Bevorzugt enthält die erfindungsgemäß verwendete Heißschmelzklebemasse Polyolefin/e mit Schmelzviskositäten, gemessen bei einer Temperatur von 170 °C, von 50 bis 30 000 mPa·s, besonders bevorzugt 100 bis 20 000 mPa·s.

Die erfindungsgemäß verwendete Heißschmelzklebemasse ist wasser- und lösungsmittelfrei und sie enthält auch keine Plastifikatoren und/oder Weichmacher.

Bevorzugt ist weiter eine Heißschmelzklebemasse, die die oben genannten Polyolefine enthält, wenn diese eine zahlenmittlere Molmasse Mₙ zwischen 500 und 20 000 g/mol, bevorzugt zwischen 800 und 10 000 g/mol, besonders bevorzugt zwischen 1 000 und 5 000 g/mol und eine gewichtsmittlere Molmasse M_{w} zwischen 1 000 und 40 000 g/mol, bevorzugt zwischen 1 600 und 30 000 g/mol und besonders bevorzugt zwischen 2 000 und 20 000 g/mol aufweisen. Die Bestimmung der Molmasse erfolgt gelpermeationschromatographisch.

In einer ebenso bevorzugten Ausführungsform der Erfindung stellen die in der erfindungsgemäß verwendeten Heißschmelzklebemasse enthaltenen Polyolefine ein Homopolymeres des Propylens oder höherer 1-Olefine oder ein Copolymeres von Olefinen, umfassend Propylen und/oder höhere 1-Olefine sowie gegebenenfalls Ethylen dar. Als höhere 1-Olefine werden vorzugsweise lineare oder verzweigte Olefine mit 4 bis 20 C-Atomen, vorzugsweise mit 4 bis 6 C-Atomen, eingesetzt. Diese Olefine können eine mit der olefinischen Doppelbindung in Konjugation stehende aromatische Substitution aufweisen. Als Beispiele für mögliche 1-Olefine seien 1-Buten, 1-Hexen, 1-Octen oder 1-Octadecen sowie Styrol genannt. Die Copolymeren bestehen vorzugsweise zu 70 bis 99,9, besonders bevorzugt zu 80 bis 99 Gew.-%, aus einer Olefinart. Zur Herstellung der Copolymeren können zwei oder mehrere der genannten Olefine eingesetzt werden.

In einer weiter bevorzugten Ausführungsform der Erfindung ist das in der erfindungsgemäß verwendeten Heißschmelzklebemasse enthaltene Polyolefin ein Copolymeres aus Propylen mit wenigstens einem oder mehreren weiteren Monomeren ausgewählt aus Ethylen und linearen oder verzweigten 1-Olefinen mit 4 bis 20 C-Atomen, vorzugsweise mit 4 bis 10 C-Atomen, wobei der Gehalt an Struktureinheiten, hervorgegangen aus Propylen, bevorzugt 70 bis 99,9, besonders bevorzugt 80 bis 99 Gew.-% beträgt.

In einer weiteren Ausführungsform ist das in der Heißschmelzklebemasse enthaltenen Polyolefin ein Copolymeres aus Ethylen und mindestens einem verzweigten oder unverzweigten 1-Olefin mit 3 bis 20 C-Atomen, wobei der Gehalt an Struktureinheiten, hervorgegangen aus Ethylen, 70 bis 99,9 Gew.-% beträgt.

In einer besonders bevorzugten Ausführungsform der Erfindung ist das in der Heißschmelzklebemasse enthaltene Polyolefin ein Copolymeres des Propylens mit 0,1 bis 30 Gew.-%, insbesondere mit 1 bis 20 Gew.-%, Ethylen.

Die erfindungsgemäß verwendete Heißschmelzklebemasse enthält das oder die Polyolefine vorzugsweise in einer Menge von 2 bis 100 Gew.-%, bevorzugt zwischen 30 und 95 Gew.-%, besonders bevorzugt zwischen 50 und 85 Gew.-%, ganz besonders bevorzugt zwischen 70 und 80 Gew.-%.

Die erfindungsgemäß verwendete Heißschmelzklebemasse enthält zusätzlich eine oder mehrere Klebekomponente(n), ausgewählt aus der Gruppe der Harze. In Betracht kommen aliphatische und cycloaliphatische oder aromatische Kohlenwasserstoffharze. Diese können durch Polymerisation bestimmter Harzölfraktionen hergestellt werden, die bei der Aufbereitung von Erdöl anfallen. Derartige Harze, die z.B. durch Hydrierung oder Funktionalisierung modifiziert werden können, sind beispielsweise unter den Handelsnamen Eastoflex, RegalREZ, Kristalex, Eastotac, Piccotac (Eastman Chemical Company) oder Escorez (ExxonMobil Chemical Company) erhältlich. Weiter kommen als Harze Polyterpen-Harze in Betracht, hergestellt durch Polymerisation von Terpenen, beispielsweise Pinen in Gegenwart von Friedel-Crafts-Katalysatoren, desgleichen hydrierte Polyterpene, Copolymere und Terpolymere von natürlichen Terpenen, beispielsweise Styrol/Terpen- oder a-Methylstyrol/Terpen-Copolymere. Weiter in Betracht kommen natürliche und modifizierte Kolophoniumharze, insbesondere Harzester, Glycerinester von Baumharzen, Pentaerithrolester von Baumharzen und Tallölharzen und deren hydrierte Derivate sowie phenol-modifizierte Pentaerithrolester von Harzen und phenol-modifizierte Terpen-Harze.

Die genannten Harze sind in der erfindungsgemäß verwendeten Heißschmelzklebemasse einzeln oder in beliebiger Kombination in Gewichtsmengen, bezogen auf das Gesamtgewicht der Heißschmelzklebemasse, im Bereich von 0 bis 60 Gew.-%, bevorzugt von 10 bis 50 Gew.%, besonders bevorzugt von 15 bis 30 Gew.-%, enthalten.

In einer ganz besonders bevorzugten Ausführungsform der Erfindung enthält die Heißschmelzklebemasse ein oder mehrere der oben beschriebenen Polyolefine und zusätzlich eine Klebekomponente, ausgewählt aus amorphen Poly-alpha-Olefinen, z.B. den Typen der Vestoplast^{®}-Reihe (Degussa) oder den "Rextac"-Typen der Fa. Huntsman, aliphatischen, cycloaliphatischen oder aromatischen Kohlenwasserstoffharzen, wie sie z.B. unter dem Handelsnamen "Escorez" bei Exxon Mobile erhältlich sind, ferner Polyisobutylen, erhältlich z.B. unter dem Handelsnamen "Oppanol" bei BASF. Weiterhin können auch andere Polyolefine, etwa Niederdruck-Polyethylene, wie sie z.B. unter dem Namen "Affinity" bei Dow Chemical verfügbar sind, enthalten sein, ferner Hochdruckpolyethylene, auch solche mit polaren Comonomeren wie z.B. EthylenVinylacetat. Die Gesamtmischung der so beschaffenen Heißschmelzklebemasse hat eine Viskosität im Bereich von 100 bis 10 000 mPa·s bei 170 °C, bevorzugt von 120 bis 9 000 mPa·s bei 170 °C, besonders bevorzugt von 130 bis 8 000 mPa·s bei 170 °C. Gegebenenfalls können zusätzlich noch Pigmente, Antioxidantien, Geruchsbinder, antimikrobielle Wirksubstanzen oder Farb- und Duftstoffe vorhanden sein.

Die erfindungsgemäße Verwendung betrifft die Herstellung verschiedenster Hygieneartikel, wie Babywindeln, Höschenwindeln, Inkontinenzprodukten, Einlagen, Binden, insbesondere zum Laminieren von Fasermaterialien wie Vliesmaterialien mit Folie.

Erfindungsgemäß wird die Heißschmelzklebemasse in Mengen von 1 bis 6 g/m², bevorzugt von 3 bis 5,5 g/m² zum Verkleben von Vlies mit Folie eingesetzt.

Als faserige Materialien, insbesondere als Vliesmaterialien, vorzugsweise für Windelvliese für die gesamte Windelkonstruktion und für Inkontinenzprodukte kommen natürliche oder synthetische Fasermaterialien, beispielsweise aus Baumwolle, Wolle, Seide, Zellulose, Leinen, Polyamid, beispielsweise Nylon oder Perlon, Polyester, beispielsweise Polyethylenterephthalat oder Polypropylenterephthalat, Polyvinylchlorid, Polypropylen, Polyethylen und auch Mischungen aus diesen Materialien in Betracht.

Die Folien können aus gängigen Materialien wie Polyethylenterephthalat, Polycarbonat, Polyethylen, Polypropylen, Polyvinylchlorid oder Polystyrol bestehen, die Heißschmelzklebemasse eignet sich aber auch für die Verbindung von faserigen Materialien mit Metalloberflächen aus Aluminium oder eisenhaltigen Metallen.

Als faserige Materialien kommen weiterhin textile Gewebe in Frage. Insbesondere können solche textilen Gewebe auch untereinander oder mit Vliesmaterial oder Folien verklebt werden.

Gegenstand der Erfindung sind weiter Hygieneartikel, bevorzugt Windeln, Inkontinenzprodukte, Einlagen und Binden, enthaltend die Heißschmelzklebemasse.

Für die Herstellung der in der erfindungsgemäß verwendeten Heißschmelzklebemasse enthaltenen Metallocen-Polyolefine werden Metallocenverbindungen der Formel alls Katalysator eingesetzt.

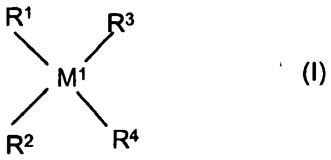

Diese Formel umfasst auch Verbindungen der Formel Ia, 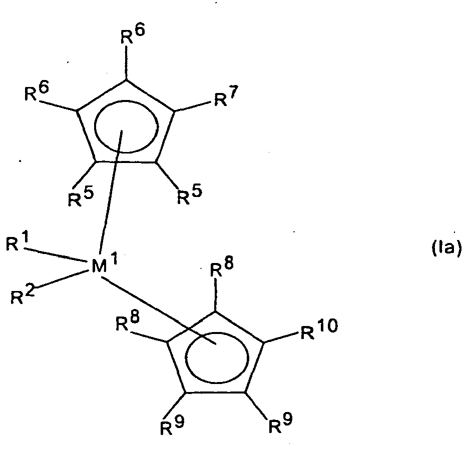 der Formel Ib, 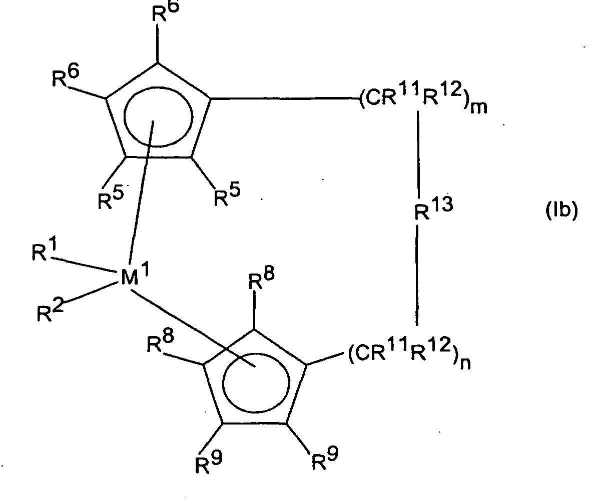 und der Formel Ic.

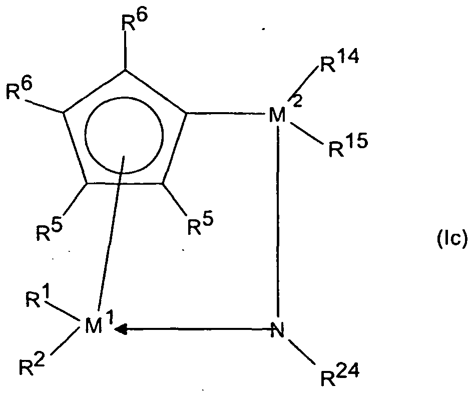

In den Formeln I, la und Ib ist M¹ ein Metall der Gruppe lVb, Vb oder Vlb des Periodensystems, beispielsweise Titan, Zirkonium, Hafnium, Vanadium, Niob, Tantal, Chrom, Molybdän, Wolfram, vorzugsweise Titan, Zirkonium und Hafnium.

R¹ und R² sind gleich oder verschieden und bedeuten ein Wasserstoffatom, eine C₁-C₁₀-, vorzugsweise C₁-C₃-Alkylgruppe, insbesondere Methyl, eine C₁-C₁₀-, vorzugsweise C₁-C₃-Alkoxygruppe, eine C₆-C₁₀-, vorzugsweise C₆-C₈-Arylgruppe, eine C₆-C₁₀-, vorzugsweise C₆-C₈-Aryloxygruppe, eine C₂-C₁₀-, vorzugsweise C₂-C₄-Alkenylgruppe, eine C₇-C₄₀-, vorzugsweise C₇-C₁₀-Arylalkylgruppe, eine C₇-C₄₀-, vorzugsweise C₇-C₁₂-Alkylarylgruppe, eine C₈-C₄₀-, vorzugsweise C₈-C₁₂-Arylalkenylgruppe oder ein Halogen-, vorzugsweise Chloratom.

R³ und R⁴ sind gleich oder verschieden und bedeuten einen ein- oder mehrkemigen Kohlenwasserstoffrest, welcher mit dem Zentralatom M¹ eine Sandwichstruktur bilden kann. Bevorzugt sind R³ und R⁴ Cyclopentadienyl, Indenyl, Tetrahydroindenyl, Benzoindenyl oder Fluorenyl, wobei die Grundkörper noch zusätzliche Substituenten tragen oder miteinander verbrückt sein können. Außerdem kann einer der Reste R³ und R⁴ ein substituiertes Stickstoffatom sein, wobei R²⁴ die Bedeutung von R¹⁷ hat und vorzugsweise Methyl, tert.-Butyl oder Cyclohexyl ist.

R⁵, R⁶, R⁷, R⁸, R⁹ und R¹⁰ sind gleich oder verschieden und bedeuten ein Wasserstoffatom, ein Halogenatom, vorzugsweise ein Fluor-, Chlor- oder Bromatom, eine C₁-C₁₀-, vorzugsweise C₁-C₄-Alkylgruppe, eine C₆-C₁₀-, vorzugsweise C₆-C₈-Arylgruppe, eine C₁-C₁₀-, vorzugsweise C₁-C₃-Alkoxygruppe, einen -NR¹⁶₂-, -SR¹⁶-, -OSiR¹⁶₃-, -SiR¹⁶₃- oder -PR¹⁶₂-Rest, worin R¹⁶ eine C₁-C₁₀, vorzugsweise C₁-C₃-Alkylgruppe oder C₆-C₁₀-, vorzugsweise C₆-C₈-Arylgruppe oder im Falle Si oder P enthaltender Reste auch ein Halogenatom, vorzugsweise Chloratom ist oder je zwei benachbarte Reste R⁵, R⁶, R⁷, R⁸, R⁹ oder R¹⁰ bilden mit den sie verbindenden C-Atomen einen Ring. Besonders bevorzugte Liganden sind die substituierten Verbindungen der Grundkörper Cyclopentadienyl, Indenyl, Tetrahydroindenyl, Benzoindenyl oder Fluorenyl.

R¹³ ist

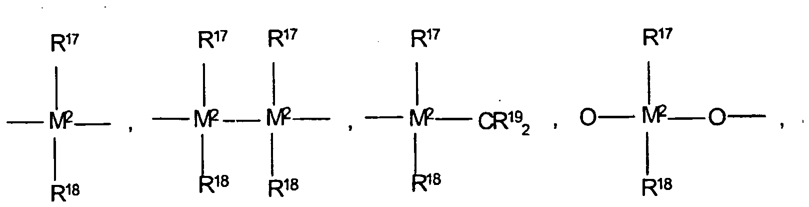

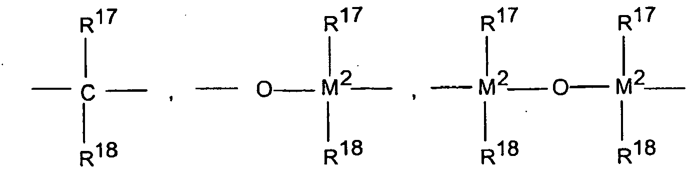

=BR¹⁷, =AIR¹⁷, -Ge-, -Sn-, -O-, -S-, =SO, =SO₂, =NR¹⁷, =CO, =PR¹⁷ oder =P(O)R¹⁷, wobei R¹⁷, R¹⁸ und R¹⁹ gleich oder verschieden sind und ein Wasserstoffatom, ein Halogenatom, vorzugsweise ein Fluor-, Chlor- oder Bromatom, eine C₁-C₃₀-, vorzugsweise C₁-C₄-Alkyl-, insbesondere Methylgruppe, eine C₁-C₁₀-Fluoralkyl-, vorzugsweise CF₃-Gruppe, eine C₆-C₁₀-Fluoraryl-, vorzugsweise Pentafluorphenylgruppe, eine C₆-C₁₀-, vorzugsweise C₆-C₈-Arylgruppe, eine C₁-C₁₀-, vorzugsweise C₁-C₄-Alkoxy-, insbesondere Methoxygruppe, eine C₂-C₁₀-, vorzugsweise C₂-C₄-Alkenylgruppe, eine C₇-C₄₀-, vorzugsweise C₇-C₁₀-Aralkylgruppe, eine C₈-C₄₀-, vorzugsweise C₈-C₁₂-Arylalkenylgruppe oder eine C₇-C₄₀-, vorzugsweise C₇-C₁₂-Alkylarylgruppe bedeuten, oder R¹⁷ und R¹⁸ oder R¹⁷ und R¹⁹ bilden jeweils zusammen mit den sie verbindenden Atomen einen Ring.

M² ist Silizium, Germanium oder Zinn, bevorzugt Silizium und Germanium. R¹³ ist vorzugsweise =CR¹⁷R¹⁸, =SiR¹⁷R¹⁸, =GeR¹⁷R¹⁸, -O-, -S-, =SO, =PR¹⁷ oder =P(O)R¹⁷.

R¹¹ und R¹² sind gleich oder verschieden und haben die für R¹⁷ genannte Bedeutung m und n sind gleich oder verschieden und bedeuten null, 1 oder 2, bevorzugt null oder 1, wobei m plus n null, 1 oder 2, bevorzugt null oder 1 ist.

R¹⁴ und R¹⁵ haben die Bedeutung von R¹⁷ und R¹⁸.

Konkrete Beispiele für geeignete Metallocene sind:
Bis(1,2,3-trimethylcyclopentadienyl)zirkoniumdichlorid,
Bis(1,2,4-trimethylcyclopentadienyl)zirkoniumdichlorid,
Bis(1,2-dimethylcyclopentadienyl)zirkoniumdichlorid,
Bis(1,3-dimethylcyclopentadienyl)zirkoniumdichlorid,
Bis(1-methylindenyl)zirkoniumdichlorid,
Bis(1-n-butyl-3-methyl-cyclopentadienyl)zirkoniumdichlorid,
Bis(2-methyl-4,6-di-i.propyl-indenyl)zirkoniumdichlorid,
Bis(2-methylindenyl)zirkoniumdichlorid,
Bis(4-methylindenyl)zirkoniumdichlorid,
Bis(5-methylindenyl)zirkoniumdichlorid,
Bis(alkylcyclopentadienyl)zirkoniumdichlorid,
Bis(alkylindenyl)zirkoniumdichlorid,
Bis(cyclopentadienyl)zirkoniumdichlorid,
Bis(indenyl)zirkoniumdichlorid,
Bis(methylcyclopentadienyl)zirkoniumdichlorid,
Bis(n-butylcyclopentadienyl)zirkoniumdichlorid,
Bis(octadecylcyclopentadienyl)zirkoniumdichlorid,
Bis(pentamethylcyclopentadienyl)zirkoniumdichlorid,
Bis(trimethylsilylcyclopentadienyl)zirkoniumdichlorid,
Biscyclopentadienylzirkoniumdibenzyl,
Biscyclopentadienylzirkoniumdimethyl,
Bistetrahyd roindenylzirkoniumdichlorid,
Dimethylsilyl-9-fluorenylcyclopentadienylzirkoniumdichlorid,
Dimethylsilyl-bis-1-(2,3,5-trimethylcyclopentadienyl)zirkoniumdichlorid,
Dimethylsilyl-bis-1-(2,4-dimethyl-cyclopentadienyl)zirkoniumdichlorid,
Dimethylsilyl-bis-1-(2-methyl-4,5-benzoindenyl)zirkoniumdichlorid,
Dimethylsilyl-bis-1-(2-methyl-4-ethylindenyl)zirkoniumdichlorid,
Dimethylsilyl-bis-1-(2-methyl-4-i-propylindenyl)zirkoniumdichlorid,
Dimethylsilyl-bis-1-(2-methyl-4-phenylindenyl)zirkoniumdichlorid,
Dimethylsilyl-bis-1-(2-methyl-indenyl)zirkoniumdichlorid,
Dimethylsilyl-bis-1-(2-methyltetrahydroindenyl)zirkoniumdichlorid,
Dimethylsilyl-bis-1-indenylzirkoniumdichlorid,
Dimethylsilyl-bis-1-indenylzirkoniumdimethyl,
Dimethylsilyl-bis-1-tetrahydroindenylzirkoniumdichlorid,
Diphenylmethylen-9-fluorenylcyclopentadienylzirkoniumdichlorid,
Diphenylsilyl-bis-1-indenylzirkoniumdichlorid,
Ethylen-bis-1-(2-methyl-4,5-benzoindenyl)zirkoniumdichlorid,
Ethylen-bis-1-(2-methyl-4-phenylindenyl)zirkoniumdichlorid,
Ethylen-bis-1-(2-methyl-tetrahydroindenyl)zirkoniumdichlorid,
Ethylen-bis-1-(4,7-dimethyl-indenyl)zirkoniumdichlorid,
Ethylen-bis-1-indenylzirkoniumdichlorid,
Ethylen-bis-1-tetrahydroindenylzirkoniumdichlorid,
Indenyl-cyclopentadienyl-zirkoniumdichlorid
Isopropyliden(1-indenyl)(cyclopentadienyl)zirkoniumdichlorid,
Isopropyliden(9-fluorenyl)(cyclopentadienyl)zirkoniumdichlorid,
Phenylmethylsilyl-bis-1-(2-methyl-indenyl)zirkoniumdichlorid,
sowie jeweils die Alkyl- oder Aryl-Derivate dieser Metallocendichloride.

Zur Aktivierung der Einzentren-Katalysatorsysteme werden geeignete Cokatalysatoren eingesetzt. Geeignete Cokatalysatoren für Metallocene der Formel I sind aluminiumorganische Verbindungen, insbesondere Alumoxane oder auch aluminiumfreie Systeme wie R²⁰ₓNH₄₋ₓBR²¹₄, R²⁰ₓPH₄₋ₓBR²¹₄, R²⁰₃CBR²¹₄ oder BR²¹₃. In diesen Formeln bedeutet x eine Zahl von 1 bis 4, die Reste R²⁰ sind gleich oder verschieden, bevorzugt gleich, und bedeuten C₁-C₁₀-Alkyl oder C₆-C₁₈-Aryl oder zwei Reste R²⁰ bilden zusammen mit dem sie verbindenden Atom einen Ring, und die Reste R²¹ sind gleich oder verschieden, bevorzugt gleich, und stehen für C₆-C₁₈-Aryl, das durch Alkyl, Haloalkyl oder Fluor substituiert sein kann. Insbesondere steht R²⁰ für Ethyl, Propyl, Butyl oder Phenyl und R²¹ für Phenyl, Pentafluorphenyl, 3,5-Bis-trifluormethylphenyl, Mesityl, Xylyl oder Tolyl.

Zusätzlich ist häufig eine dritte Komponente erforderlich, um einen Schutz von polaren Katalysator-Giften aufrecht zu erhalten. Hierzu sind aluminiumorganische Verbindung wie z.B. Triethylaluminium, Tributylaluminium und andere sowie Mischungen aus diesen geeignet.

Je nach Verfahren können auch geträgerte Einzentren-Katalysatoren zur Verwendung kommen. Bevorzugt sind Katalysatorsysteme, bei welchen die Restgehalte von Trägermaterial und Cokatalysator eine Konzentration von 100 ppm im Produkt nicht überschreiten.

Verfahren zur Herstellung derartiger Polyolefine sind beispielsweise im Stand der Technik wie EP-A-0 321 851, EP-A-0 321 852, EP-A-0 384 264, EP-A-0 571 882 und EP-A-0 890 584 beschrieben.

### Ausführungsbeispiele

Die nachfolgenden Beispiele sollen die Erfindung näher erläutern, ohne sie jedoch auf die konkret angegebenen Ausführungsformen zu beschränken. Prozentangaben sind, wenn nicht anders angegeben, als Gewichtsprozente zu verstehen.

Die Schmelzviskositäten wurden nach DIN 53019 mit einem Rotationsviskosimeter, die Tropfpunkte nach ASTM D3954, die Erweichungspunkte Ring/Kugel nach ASTM D3104, bestimmt. Das Molmassengewichtsmittel M_{w} und das Molmassenzahlenmittel Mₙ wurden durch Gelpermeationschromatographie bei einer Temperatur von 135 °C in 1,2-Dichlorbenzol ermittelt.

Die erfindungsgemäß eingesetzten, in Tabelle 1 aufgeführten Polyolefine 1 bis 4 wurden nach im Stand der Technik gemäß EP 0 384 264 bzw. EP 0 571 882 angegebenen Verfahren hergestellt.

**Tabelle 1: Eingesetzte Polyolefine**

| | Polyolefin 1 | Polyolefin 2 | Polyolefin 3 | Polyolefin 4 |
|---|---|---|---|---|
| Herstellung gemäß | EP 0 384 264 allgem.Vorschrift Bsp. 1-16¹⁾ | EP 0 384 264 allgem.Vorschrift Bsp. 1-16²⁾ | EP 0 571 882 Bsp. 3 | EP 0 384 264 allgem.Vorschrift Bsp. 1-16³⁾ |
| Typ | Propylen-Ethylen-Copolymer | Propylen-Ethylen-Copolymer | Propylen-Homopolymer | Propylen-Ethylen-Copolymer |
| Erweichungspunkt (°C) | 83⁴⁾ | 88 | 145⁴⁾ | 106 |
| Viskosität bei 170°C (mPa-s) | 180 | 11500 | 101 | 4300 |
| Mₙ | 2760 | 8250 | 1980 | 5320 |
| M_{w} | 6320 | 19110 | 3900 | 12030 |

| | | | | |
|---|---|---|---|---|
| ¹⁾ Polymerisationsdaten: Ethyleneinsatz gesamt 400 g, Polymerisationstemp. 75 °C ²⁾ Ethyleneinsatz gesamt 350 g, Polymerisationstemp. 65 °C ³⁾ Ethyleneinsatz gesamt 200 g, Polymerisationstemp. 50 °C ⁴⁾ Tropfpunkt | | | | |

Die vorstehend näher bezeichneten Polyolefine 1 bis 4 wurden mit den in Tabelle 2 aufgeführten zusätzlichen Komponenten in den angegebenen Mischungsverhältnissen aufgeschmolzen und bei einer Temperatur von 170 °C gemischt.

**Tabelle 2: Beispielrezepturen und Vergleichsrezepturen (Angaben in Gew.-%) und resultierende Initial Peel Werte in N/mm², gemessen bei 37 °C**

| Beispiel | 1 | 2 | 3 | 4 | 5 |
|---|---|---|---|---|---|
| Polyolefin 1 | 80 | | | | |
| Polyolefin 2 | | 80 | | | |
| Polyolefin 3 | | | 5 | | |
| Polyolefin 4 | | | 75 | 80 | |
| Affinity GA-1900 | | | | | 60 |
| Escorez 5637 | 20 | 20 | 20 | 20 | 40 |
| Oppanol B11 | | | | | |
| Vestoplast 828 | | | | | |
| Initial Peel | - | 364 | 544 | - | 205 |
| | | | | | |

| Beispiel | 6 | 7 | 8 | 9 | 10 |
|---|---|---|---|---|---|
| Comparison I SIS based | 100 | | | | |
| Comparison II SIS based | | 100 | | | |
| Huntsman RT-2730 | | | 100 | | |
| Polyolefin 3 | | | | 70 | 30 |
| Escorez 5400 | | | | | 40 |
| Escorez 5637 | | | | 20 | |
| Oppanol B11 | | | | 10 | |
| Vestoplast 828 | | | | | 30 |
| Initial Peel | 192 | 205 | 166 | 473 | 392 |

Zur Ermittlung der Peel Werte der getesteten Heißschmelzklebemassen wurde Vliesmaterial - BBA Style 717D (16,9 gsm) mit einer Polyethylenfolie Clopay DH-203 PE jeweils mit der Heißschmelzklebemasse gemäß den Beispielrezepturen 1 bis 10 bei 25 °C verklebt, wobei jeweils 6,2 g/m² Heißschmelzklebemasse auf das Vliesmaterial aufgesprüht und dann das besprühte Vlies und die Folie unverzüglich mit Walzen verpresst wurden. Die Proben wurden nach 24 h bei einer Lagerungtemperatur von 25 °C unter Normbedingungen mit einer Geschwindigkeit von 12 inches/min und 180° peel Winkel bei 25 °C geschält.

Die Prüfergebnisse zeigen anhand der initial peel Werte eine z. T. sehr starke Zunahme der Klebekräfte der Heißschmelzklebemassen, enthaltend die erfindungsgemäße eingesetzten Polyolefine, im Vergleich zu Heißschmelzklebemassen des Standes der Technik (siehe Beispiele 5, 6, 7 und 8). Folglich kann auch beim Einsatz der erfindungsgemäßen Heißschmelzklebemassen in Mengen unterhalb von 6 g/m² eine ausreichende Klebewirkung erzielt werden.

Chemische Bezeichnung der eingesetzten Handelsprodukte:
- Affinity GA-1900: Polyethylen (Dow Chemical Corp.)
- Escorez 5637: Kohlenwasserstoffharz (Exxon Mobile)
- Escorez 5400: Kohlenwasserstoffharz (Exxon Mobile)
- Oppanol B11: Polyisobuten (BASF)
- Vestoplast 828: amorphes Poly-alpha-olefin (Degussa)
- Huntsman RT-2730: amorphes Poly-alpha-olefin (Huntsmann)
- Comparison I SIS based: Schmelzkleberrezeptur bestehend aus Styrol-Isopren-Blockcopolymer, Öl, Harz (HB Fuller)
- Comparison II SIS based: Schmelzkleberrezeptur bestehend aus Styrol-Isopren-Blockcopolymer, Öl, Harz (National Starch)

## Patentansprüche

1. Verwendung einer Heissschmelzklebemasse zum Verkleben von faserigen Materialien untereinander oder mit glatten Substratoberflächen bei der Herstellung von Hygieneartikeln, **dadurch gekennzeichnet, dass** die Heissschmelzklebemasse mindestens ein Polyolefin enthält, das durch Polymerisation in Gegenwart von Metallocen als Katalysator hergestellt wurde und das einen Erweichungspunkt Ring/Kugel zwischen 50 und 165 °C und eine Schmelzviskosität, gemessen bei einer Temperatur von 170 °C, zwischen 20 und 40 000 mPa·s aufweist.

2. Verwendung einer Heissschmelzklebemasse nach Anspruch 1, **dadurch gekennzeichnet, dass** die Heissschmelzklebemasse ein oder mehrere Polyolefin/e enthält, das oder die eine Glasübergangstemperatur T_{g} von maximal -10 °C und einen Schmelzflussindex MFI von mehr als 30 g/10 min aufweisen, gemessen nach ISO 1133 bei einer Temperatur von 190 °C und einem Auflagegewicht von 2,16 kg.

3. Verwendung einer Heissschmelzklebemasse nach Anspruch 1 oder 2, **dadurch gekennzeichnet, dass** die Heissschmelzklebemasse ein oder mehrere Polyolefine mit einer Schmelzviskosität, gemessen bei einer Temperatur von 170 °C, im Bereich von 50 bis 30 000 mPa·s, besonders bevorzugt 100 bis 20 000 mPa·s, enthält.

4. Verwendung einer Heissschmelzklebemasse nach einem oder nach mehreren der Ansprüche 1 bis 3, **dadurch gekennzeichnet, dass** die Heissschmelzklebemasse wasser- und lösungsmittelfrei ist und dass sie frei ist von Plastifikatoren und/oder Weichmachern.

5. Verwendung einer Heissschmelzklebemasse nach einem oder nach mehreren der Ansprüche 1 bis 4, **dadurch gekennzeichnet, dass** die Heissschmelzklebemasse Polyolefine enthält, die eine zahlenmittlere Molmasse Mₙ zwischen 500 und 20 000 g/mol besitzen, bevorzugt zwischen 800 und 10 000 g/mol, besonders bevorzugt zwischen 1 000 und 5 000 g/mol, und eine gewichtsmittlere Molmasse M_{w} zwischen 1 000 und 40 000 g/mol, bevorzugt zwischen 1 600 und 30 000 g/mol, besonders bevorzugt zwischen 2 000 und 20 000 g/mol.

6. Verwendung einer Heissschmelzklebemasse nach einem oder nach mehreren der Ansprüche 1 bis 5, **dadurch gekennzeichnet, dass** die Heissschmelzklebemasse Polyolefine enthält, die Homopolymere des Propylens oder höherer 1-Olefine oder Copolymere von Olefinen, umfassend Propylen und/oder höhere 1-Olefine sowie gegebenenfalls Ethylen darstellen, wobei als höhere 1-Olefine lineare oder verzweigte Olefine mit 4 bis 20 C-Atomen, vorzugsweise mit 4 bis 6 C-Atomen, gelten.

7. Verwendung einer Heissschmelzklebemasse nach einem oder nach mehreren der Ansprüche 1 bis 5, **dadurch gekennzeichnet, dass** die Heissschmelzklebemasse Polyolefine enthält, die Copolymere aus Propylen mit wenigstens einem oder mit mehreren weiteren Monomeren ausgewählt aus Ethylen und linearen oder verzweigten 1 -Olefinen mit 4 bis 20 C-Atomen, vorzugsweise mit 4 bis 10 C-Atomen, darstellen, wobei der Gehalt an Struktureinheiten, hervorgegangen aus Propylen, bevorzugt 70 bis 99,9 Gew.-%, besonders bevorzugt 80 bis 99 Gew.-%, beträgt.

8. Verwendung einer Heissschmelzklebemasse nach einem oder nach mehreren der Ansprüche 1 bis 5, **dadurch gekennzeichnet, dass** die Heissschmelzklebemasse als Polyolefin ein Copolymeres aus Ethylen und mindestens einem verzweigten oder unverzweigten 1-Olefin mit 3 bis 20 C-Atomen enthält, wobei der Gehalt an Struktureinheiten, hervorgegangen aus Ethylen, 70 bis 99,9 Gew.-% beträgt.

9. Verwendung einer Heissschmelzklebemasse nach einem oder nach mehreren der Ansprüche 1 bis 8, **dadurch gekennzeichnet, dass** die Heissschmelzklebemasse das oder die Polyolefin/e in einer Menge von 2 bis 100 Gew.-% enthält, bevorzugt von 30 bis 95 Gew.-%, besonders bevorzugt von 50 bis 85 Gew.-%, ganz besonders bevorzugt von 70 bis 80 Gew.-%.

10. Verwendung einer Heissschmelzklebemasse nach einem oder nach mehreren der Ansprüche 1 bis 9, **dadurch gekennzeichnet, dass** die Heissschmelzklebemasse zusätzlich eine oder mehrere Klebekomponente(n) enthält, ausgewählt aus der Gruppe der Harze enthaltend aliphatische und cycloaliphatische oder aromatische Kohlenwasserstoffharze, Polyterpen-Harze, hergestellt durch Polymerisation von Terpenen in Gegenwart von Friedel-CraftsKatalysatoren, hydrierte Polyterpene, Copolymere und Terpolymere von natürlichen Terpenen, Styrol/Terpenoder [alpha]-Methylstyrol/Terpen-Copolymere, natürliche und modifizierte Kolophoniumharze, insbesondere Harzester, Glycerinester von Baumharzen, Pentaerithrolester von Baumharzen und Tallölharzen sowie deren hydrierte Derivate sowie phenol-modifizierte Pentaerithrolester von Harzen und phenol-modifizierte Terpen-Harze.

11. Verwendung einer Heissschmelzklebemasse nach einem oder nach mehreren der Ansprüche 1 bis 10, **dadurch gekennzeichnet, dass** die Heissschmelzklebemasse mindestens eines der Harze oder eine beliebige Kombination davon in einer Menge, bezogen auf das Gesamtgewicht der Heissschmelzklebemasse, im Bereich von 0 bis 60 Gew.-% enthält, bevorzugt von 10 bis 50 Gew.-%, besonders bevorzugt von 15 bis 30 Gew.-%.

12. Verwendung einer Heissschmelzklebemasse nach einem oder nach mehreren der Ansprüche 1 bis 11 , **dadurch gekennzeichnet, dass** die Heissschmelzklebemasse ein Polyolefin, bevorzugt Polyethylen, und zusätzlich eine Klebekomponente, ausgewählt aus amorphen Poly-alpha-Olefinen, aliphatischen, cycloaliphatischen oder aromatischen Kohlenwasserstoffharzen enthält und dass sie eine Viskosität im Bereich von 100 bis 10 000 mPa·s bei 170 °C besitzt, bevorzugt von 120 bis 9 000 mPa·s bei 170 °C, besonders bevorzugt von 130 bis 8 000 mP·as bei 170 °C.

13. Verwendung einer Heissschmelzklebemasse nach einem oder nach mehreren der Ansprüche 1 bis 12, **dadurch gekennzeichnet, dass** die Heissschmelzklebemasse zusätzlich noch Pigmente, Antioxidantien, Geruchsbinder, antimikrobielle Wirksubstanzen und/oder Färb- und Duftstoffe in jeweils wirksamer Menge enthält.

14. Verwendung einer Heissschmelzklebemasse nach einem oder nach mehreren der Ansprüche 1 bis 13 bei der Herstellung von Babywindeln, Höschenwindeln, Inkontinenzprodukten, Slipeinlagen oder Binden.

15. Verwendung einer Heissschmelzklebemasse nach Anspruch 14 in einer Menge von 1 bis 6 g/m², bevorzugt von 3 bis 5,5 g/m², zum Verkleben von Vlies mit Folie.

16. Verwendung einer Heissschmelzklebemasse nach Anspruch 14 oder 15, bei der als Vliesmaterialien Fasermaterialien aus Baumwolle, aus Wolle, aus Seide, aus Zellulose, aus Leinen, aus Polyamid, aus Polyester, beispielsweise aus Polyethylentherephthalat oder Polypropylentherephthalat, aus Polyvinylchlorid, aus Polypropylen, aus Polyethylen und aus Mischungen von diesen Materialien eingesetzt werden.

17. Verwendung einer Heissschmelzklebemasse nach einem oder nach mehreren der Ansprüche 14 bis 16, bei der als Folien eine Folie auf Basis von Polyethylenterephthalat, von Polycarbonat, von Polyethylen, von Polypropylen, von Polyvinylchlorid oder von Polystyrol eingesetzt wird.

18. Hygieneartikel, vorzugsweise Höschenwindel, Babywindel, Inkontinenzprodukt, Slipeinlage und/oder Damenbinde,hergestellt unter der Verwendung einer Heissschmelzklebemasse nach einem oder nach mehreren der Ansprüche 1 bis 17.

## Claims

1. The use of a hot melt adhesive for adhesively bonding fibrous materials to one another or to smooth substrate surfaces in the production of hygiene articles, wherein the hot melt adhesive contains at least one polyolefin which has been prepared by polymerization in the presence of metallocene as catalyst and has a ring/ball softening point in the range from 50 to 165°C and a melt viscosity measured at a temperature of 170°C in the range from 20 to 40 000 mPa·s.

2. The use of a hot melt adhesive as claimed in claim 1, wherein the hot melt adhesive contains one or more polyolefin(s) having a glass transition temperature T_{g} of not more than -10°C and a melt flow index MFI of greater than 30 g/10 min, measured in accordance with ISO 1133 at a temperature of 190°C and under a load of 2.16 kg.

3. The use of a hot melt adhesive as claimed in claim 1 or 2, wherein the hot melt adhesive contains one or more polyolefins having a melt viscosity measured at a temperature of 170°C in the range from 50 to 30 000 mPa·s, particularly preferably from 100 to 20 000 mPa·s.

4. The use of a hot melt adhesive as claimed in one or more of claims 1 to 3, wherein the hot melt adhesive is water- and solvent-free and is free of plasticators and/or plasticizers.

5. The use of a hot melt adhesive as claimed in one or more of claims 1 to 4, wherein the hot melt adhesive contains polyolefins having a number average molar mass Mₙ in the range from 500 to 20 000 g/mol, preferably from 800 to 10 000 g/mol, particularly preferably from 1000 to 5000 g/mol, and a weight average molar mass M_{w} in the range from 1000 to 40 000 g/mol, preferably from 1600 to 30 000 g/mol, particularly preferably from 2000 to 20 000 g/mol.

6. The use of a hot melt adhesive as claimed in one or more of claims 1 to 5, wherein the hot melt adhesive contains polyolefins which are homopolymers of propylene or higher 1-olefins or copolymers of olefins comprising propylene and/or higher 1-olefins and also, if appropriate, ethylene, with higher 1-olefins being considered to be linear or branched olefins having from 4 to 20 carbon atoms, preferably from 4 to 6 carbon atoms.

7. The use of a hot melt adhesive as claimed in one or more of claims 1 to 5, wherein the hot melt adhesive contains polyolefins which are copolymers of propylene with at least one or more further monomers selected from among ethylene and linear or branched 1-olefins having from 4 to 20 carbon atoms, preferably from 4 to 10 carbon atoms, with the content of structural units derived from propylene preferably being from 70 to 99.9% by weight, particularly preferably from 80 to 99% by weight.

8. The use of a hot melt adhesive as claimed in one or more of claims 1 to 5, wherein the hot melt adhesive contains a copolymer of ethylene and at least one branched or unbranched 1-olefin having from 3 to 20 carbon atoms, with the content of structural units derived from ethylene being from 70 to 99.9% by weight, as polyolefin.

9. The use of a hot melt adhesive as claimed in one or more of claims 1 to 8, wherein the hot melt adhesive contains the polyolefin(s) in an amount of from 2 to 100% by weight, preferably from 30 to 95% by weight, particularly preferably from 50 to 85% by weight, very particularly preferably from 70 to 80% by weight.

10. The use of a hot melt adhesive as claimed in one or more of claims 1 to 9, wherein the hot melt adhesive additionally contains one or more tackifier(s) selected from the group of resins consisting of aliphatic and cycloaliphatic or aromatic hydrocarbon resins, polyterpene resins prepared by polymerization of terpenes in the presence of Friedel-Crafts catalysts, hydrogenated polyterpenes, copolymers and terpolymers of natural terpenes, styrene-terpene or [alpha]-methylstyrene-terpene copolymers, natural and modified rosins, in particular resin esters, glyceryl esters of tree resins, pentaerithrityl esters of tree resins and tall oil resins and hydrogenated derivatives thereof and also phenol-modified pentaerithrityl esters of resins and phenol-modified terpene resins.

11. The use of a hot melt adhesive as claimed in one or more of claims 1 to 10, wherein the hot melt adhesive contains at least one of the resins or any combination thereof in an amount, based on the total weight of the hot melt adhesive, in the range from 0 to 60% by weight, preferably from 10 to 50% by weight, particularly preferably from 15 to 30% by weight.

12. The use of a hot melt adhesive as claimed in one or more of claims 1 to 11, wherein the hot melt adhesive contains a polyolefin, preferably polyethylene, and additionally a tackifier selected from among amorphous poly-alpha-olefins, aliphatic, cycloaliphatic or aromatic hydrocarbon resins and has a viscosity in the range from 100 to 10 000 mPa·s at 170°C, preferably from 120 to 9000 mPa·s at 170°C, particularly preferably from 130 to 8000 mPa·s at 170°C.

13. The use of a hot melt adhesive as claimed in one or more of claims 1 to 12, wherein the hot melt adhesive additionally contains pigments, antioxidants, odor binders, antimicrobial agents and/or colorants and fragrances in an effective amount in each case.

14. The use of a hot melt adhesive as claimed in one or more of claims 1 to 13 in the production of diapers, panty diapers, incontinence products, panty liners or sanitary towels.

15. The use of a hot melt adhesive as claimed in claim 14 in an amount of from 1 to 6 g/m², preferably from 3 to 5.5 g/m², for adhesively bonding nonwoven to film.

16. The use of a hot melt adhesive as claimed in claim 14 or 15, wherein fiber materials composed of cotton, wool, silk, cellulose, linen, polyamide, polyester, for example polyethylene terephthalate or polypropylene terephthalate, polyvinyl chloride, propylene, polyethylene or mixtures of these materials are used as nonwoven materials.

17. The use of a hot melt adhesive as claimed in one or more of claims 14 to 16, wherein a film based on polyethylene terephthalate, polycarbonate, polyethylene, polypropylene, polyvinyl chloride or polystyrene is used as film.

18. A hygiene article, preferably a panty diaper, diaper, incontinence product, panty liner or sanitary towel, produced using a hot melt adhesive as claimed in one or more of claims 1 to 17.

## Revendications

1. Utilisation d'une masse adhésive thermofusible pour le collage de matériaux fibreux les uns aux autres ou sur des surfaces de substrats lisses lors de la fabrication d'articles hygiéniques, **caractérisée en ce que** la masse adhésive thermofusible contient au moins une polyoléfine qui a été préparée par polymérisation en présence de métallocène comme catalyseur et présente un point de ramollissement anneau/cône entre 50 et 165°C ainsi qu'une viscosité en masse fondue, mesurée à une température de 170°C, entre 20 et 40 000 mPa·s.

2. Utilisation d'une masse adhésive thermofusible selon la revendication 1, **caractérisée en ce que** la masse adhésive thermofusible contient une ou plusieurs polyoléfine(s), qui présente(nt) une température de transition vitreuse Tg d'au maximum -10°C et un indice de fluidité à chaud MFI de plus de 30 g/10 min, mesuré selon la norme ISO 1133 à une température de 190°C et sous une charge de 2,16 kg.

3. Utilisation d'une masse adhésive thermofusible selon la revendication 1 ou 2, **caractérisée en ce que** la masse adhésive thermofusible contient une ou plusieurs polyoléfine(s) présentant une viscosité en masse fondue, mesurée à une température de 170°C, dans la plage de 50 à 30 000 mPa.s, de manière particulièrement préférée de 100 à 20 000 mPa.s.

4. Utilisation d'une masse adhésive thermofusible selon l'une ou plusieurs des revendications 1 à 3, **caractérisée en ce que** la masse adhésive thermofusible est exempte d'eau et de solvant et **en ce qu'**elle est exempte de plastifiants et/ou d'agents de ramollissement.

5. Utilisation d'une masse adhésive thermofusible selon l'une ou plusieurs des revendications 1 à 4, **caractérisée en ce que** la masse adhésive thermofusible contient des polyoléfines, qui présentent une masse molaire numérique moyenne Mₙ entre 500 et 20 000 g/mole, de préférence entre 800 et 10 000 g/mole, de manière particulièrement préférée entre 1000 et 5000 g/mole et une masse molaire pondérale moyenne M_{w} entre 1000 et 40 000 g/mole, de préférence entre 1600 et 30 000 g/mole et de manière particulièrement préférée entre 2000 et 20 000 g/mole.

6. Utilisation d'une masse adhésive thermofusible selon l'une ou plusieurs des revendications 1 à 5, **caractérisée en ce que** la masse adhésive thermofusible contient des polyoléfines, qui représentent des homopolymères du propylène ou de 1-oléfines supérieures ou des copolymères d'oléfines, comprenant du propylène et/ou des 1-oléfines supérieures ainsi que le cas échéant de l'éthylène, les 1-oléfines supérieures étant des oléfines linéaires ou ramifiées comprenant 4 à 20 atomes de carbone, de préférence 4 à 6 atomes de carbone.

7. Utilisation d'une masse adhésive thermofusible selon l'une ou plusieurs des revendications 1 à 5, **caractérisée en ce que** la masse adhésive thermofusible contient des polyoléfines qui sont des copolymères de propylène avec au moins un ou plusieurs autres monomères choisis parmi l'éthylène et les 1-oléfines linéaires ou ramifiées comprenant 4 à 20 atomes de carbone, de préférence 4 à 10 atomes de carbone, la teneur en unités structurelles dérivées du propylène étant de préférence de 70 à 99,9, de manière particulièrement préférée de 80 à 99% en poids.

8. Utilisation d'une masse adhésive thermofusible selon l'une ou plusieurs des revendications 1 à 5, **caractérisée en ce que** la masse adhésive thermofusible contient, comme polyoléfine, un copolymère d'éthylène et d'au moins une 1-oléfine linéaire ou ramifiée comprenant 3 à 20 atomes de carbone, la teneur en unités structurelles dérivées de l'éthylène étant de 70 à 99,9% en poids.

9. Utilisation d'une masse adhésive thermofusible selon l'une ou plusieurs des revendications 1 à 8, **caractérisée en ce que** la masse adhésive thermofusible contient la ou les polyoléfine(s) en une quantité de 2 à 100% en poids, de préférence de 30 à 95% en poids, de manière particulièrement préférée de 50 à 85% en poids, de manière tout particulièrement préférée de 70 à 80% en poids.

10. Utilisation d'une masse adhésive thermofusible selon l'une ou plusieurs des revendications 1 à 9, **caractérisée en ce que** la masse adhésive thermofusible contient en outre un ou plusieurs composant(s) adhésif(s), choisi(s) dans le groupe des résines contenant les résines hydrocarbonées aliphatiques et cycloaliphatiques ou aromatiques, les résines polyterpéniques, préparées par polymérisation de terpènes, en présence de catalyseurs de Friedel-Craft, les polyterpènes hydrogénés, les copolymères et les terpolymères de terpènes naturels, les copolymères de styrène/terpène ou d'alpha-méthylstyrène/terpène, les résines de colophonium naturelles et modifiées, en particulier les esters de résine, les esters de glycérol de colophanes, les esters de pentaérythritol de colophanes et les résines de tall oil ainsi que leurs dérivés hydrogénés, ainsi que les esters de pentaérythritol modifiés par phénol de résines et les résines terpéniques modifiées par phénol.

11. Utilisation d'une masse adhésive thermofusible selon l'une ou plusieurs des revendications 1 à 10, **caractérisée en ce que** la masse adhésive thermofusible contient au moins une des résines ou une combinaison quelconque de celles-ci en une quantité, par rapport au poids total de la masse adhésive thermofusible, dans la plage de 0 à 60% en poids, de préférence de 10 à 50% en poids, de manière particulièrement préférée de 15 à 30% en poids.

12. Utilisation d'une masse adhésive thermofusible selon l'une ou plusieurs des revendications 1 à 11, **caractérisée en ce que** la masse adhésive thermofusible contient une polyoléfine, de préférence le polyéthylène, et en outre un composant adhésif, choisi parmi les poly-alpha-oléfines amorphes, les résines hydrocarbonées aliphatiques, cycloaliphatiques ou aromatiques et **en ce qu'**elle présente une viscosité dans la plage de 100 à 10 000 mPa.s à 170°C, de préférence de 120 à 9000 mPa.s à 170°C, de manière particulièrement préférée de 130 à 8000 mPa.s à 170°C.

13. Utilisation d'une masse adhésive thermofusible selon l'une ou plusieurs des revendications 1 à 12, **caractérisée en ce que** la masse adhésive thermofusible contient en outre encore des pigments, des antioxydants, des liants d'odeurs, des substances actives antimicrobiennes et/ou des colorants et des parfums à chaque fois en une quantité active.

14. Utilisation d'une masse adhésive thermofusible selon l'une ou plusieurs des revendications 1 à 13 lors de la fabrication de couches pour bébés, de couches, de produits d'incontinence, de protège-slips ou de serviettes hygiéniques.

15. Utilisation d'une masse adhésive thermofusible selon la revendication 14 en une quantité de 1 à 6 g/m², de préférence de 3 à 5,5 g/m², pour le collage d'une nappe avec une feuille.

16. Utilisation d'une masse adhésive thermofusible selon la revendication 14 ou 15, dans laquelle on utilise comme matériaux de nappe des matériaux fibreux en coton, en laine, en soie, en cellulose, en lin, en polyamide, en polyester, par exemple en poly(téréphtalate d'éthylène) ou en poly(téréphtalate de propylène), en poly(chlorure de vinyle), en polypropylène, en polyéthylène et en mélanges de ces matériaux.

17. Utilisation d'une masse adhésive thermofusible selon l'une ou plusieurs des revendications 14 à 16 dans laquelle on utilise comme feuilles une feuille à base de poly(téréphtalate d'éthylène), de polycarbonate, de polyéthylène, de polypropylène, de poly(chlorure de vinyle) ou de polystyrène.

18. Article hygiénique, de préférence couche, couche pour bébés, produit d'incontinence, protège-slips et/ou serviette hygiénique, fabriqué en utilisant une masse adhésive thermofusible selon l'une ou plusieurs des revendications 1 à 17.
